# EUROPEAN PATENT SPECIFICATION

(11) **EP 0 680 301 B1**
(45) Date of publication and mention of the grant of the patent: **24.09.1997**
(21) Application number: 92922472.3
(22) Date of filing: 27.10.1992
(51) Int. Cl.: A61F 13/15

(54) **DISPOSABLE DIAPER WITH FULLY ELASTICIZED WAIST OPENING**
WEGWERFWINDEL MIT VOLLELASTIFIZIERTEM TAILLENBUND
COUCHE-CULOTTE JETABLE A TAILLE ENTIEREMENT ELASTIQUE

(43) Date of publication of application: 08.11.1995
(73) Proprietor: PROCTER & GAMBLE FAR EAST, INC., Kobe-shi, Hyogo 658 (JP)
(72) Inventor: ROLLAG, Keith W., Ashiya-shi, Hyogo 659 (JP); YAP, Shyi Earn, Nada-ku, Kobe-shi, Hyogo 657 (JP)
(74) Representative: Bottema, Johan Jan
(86) International application number: JP9201389
(87) International publication number: WO9409736

(56) References cited:
- EP-A- 0 460 467
- WO-A-85/05254
- JP-U- 3 107 919

## Description

### BACKGROUND OF THE INVENTION

### Field of the Invention

The present invention relates to a disposable pull-on diaper having elastic side panels providing a fully elasticized waist opening.

### Related Art

Disposable diapers are well known articles of manufacture which are designed to be worn by infants and incontinent persons. Disposable diapers are worn about the lower torso of the user and are intended to absorb and contain voided urine and feces thereby preventing the soiling, wetting, or similar contamination of articles (e.g., clothing, bedding, other persons, etc.) which may come into contact with such diaper in use.

There are numerous executions of a disposable diaper available in the art which generally comprises a "chassis" comprising an absorbent core encased between a liquid permeable topsheet and a liquid impermeable backsheet. Additionally, numerous disposable diaper are available in the art which feature elastic means formed along that portion of the disposable diaper which contacts the wearer's waist to thereby provide elasticized waist opening for such diaper in use.

For example, WO85/05254 (Boussac Saint Freres, B.S.F.), published on December 5, 1985 discloses a pant made by attaching pre-stretched longitudinally directed elastic to the ends of each lateral side of the chassis and making partial cuts in the chassis inboard of the elastic. When the elastic is relaxed, the pant is formed. The waist opening of the pant is comprised of the two lengths of the elastics at the sides, and the front and the back end of the pant chassis. A disposable pant-style diaper featuring a partially elasticized waist opening is shown in Japanese Utility Model Laid-Open Publication No. 107919/1991 (Kamata), published on November 6, 1991. This diaper comprises an absorbent part and an elastic gather divided into two parts by a longitudinal slit. The outboard lateral part of the pre-stretched gather is attached to a lateral edge of the absorbent part and ends of the gather are attached to ends of the absorbent part. The inboard edge of the gather is unattached, so that the two parts of the gathering work as a waist gathering and a leg gathering, respectively.

None of the foregoing diapers are specifically directed to a disposable diaper having a fully elasticized waist opening and the problems of providing a broader range of fit characteristics associated with such diapers.

Japanese Patent Laid-Open Publication No. 28365/1992 (UNICHARM), published on January 30, 1992, is directed to a disposable diaper having an elasticized waist opening members attached to the backsheet side, and achieves an improved fit of the diaper about the waist as the diaper is worn. The diaper must be turned inside out prior to actual use, and this may cause for the user inconvenience or a perception of uncleanliness since the skin-contacting surface is exposed, and complications in the manufacture.

### SUMMARY OF THE INVENTION

It is therefore an object of the present invention to provide a disposable diaper having a fully elasticized waist opening with an improved fit of the diaper on the waist and leg openings.

A further object of the present invention is to provide a disposable diaper which the user can pull on without turning the diaper inside out.

A still further object of the present invention is to provide a method for preparing a pull-on pants-style diaper.

According to the first aspect of the present invention we provide a disposable pull-on diaper having a waist opening and leg openings, comprising, in a flat state:
an absorbent chassis comprising a liquid-pervious topsheet, a liquid-impervious backsheet and an absorbent core having a pair of side edges interposed between the topsheet and the backsheet, the chassis having a pair of sides, a front edge, a back edge, and a longitudinal centerline which divides the chassis into halves, the chassis being divided into a crotch region, a front waist region having the front edge and a back waist region having the back edge wherein the front waist region and the back waist region extend oppositely from the crotch region along the longitudinal centerline;
at least one elastic panel attached to a wearer-facing surface of each half of the chassis, each elastic panel having a waist opening edge and an opposing leg opening edge, the waist opening edge of each elastic panel extending at least to the longitudinal centerline of the chassis, each elastic panel being attached to the chassis along attachment lines which comprise a front attachment line connecting a point near the longitudinal centerline in the front waist region of the chassis to a point on each side of the chassis and a back attachment line connecting a point near the longitudinal centerline in the back waist region of the chassis to a point on each side of the chassis; and
a leg opening in a region along the leg opening edge of the elastic panel;
wherein the elastic panels form completely the waist opening.

According to the second aspect of the present invention we provide a method for preparing a disposable pull-on diaper having a waist opening and leg openings, having a liquid-pervious topsheet, a liquid-impervious backsheet, an absorbent core, and a pair of elastic panels each having a waist opening edge and an opposing leg opening edge, comprising the steps of:
- interposing and affixing the absorbent core between the topsheet and the backsheet, to assemble an absorbent chassis which has a pair of sides and a longitudinal centerline dividing the chassis into halves and which comprises the topsheet, the backsheet and the absorbent core interposed therebetween wherein the absorbent chassis is divided into a crotch region, a front waist region and a back waist region and the front waist region and the back waist region extend oppositely from the crotch region along the longitudinal centerline;
- registering a pre-stretched elastic panel with each half of the chassis in such a manner that the waist opening edge of the elastic panel extends at least along the longitudinal centerline of the chassis; and
- attaching the pre-stretched elastic panel to the chassis along attachment lines which comprise a front attachment line connecting a point near the longitudinal centerline in the front waist region of the chassis to a point on each side of the chassis and a back attachment line connecting a point near the longitudinal centerline in the back waist region of the chassis to a point on each side of the chassis whereby the waist opening edges of the elastic panels form completely the waist opening of the diaper.

According to the third aspect of the present invention we provide a method for preparing a disposable pull-on diaper having a waist opening and leg openings, having a liquid-pervious topsheet, a liquid-impervious backsheet, an absorbent core, and a pair of pre-stretched elastic panels each having a waist opening edge and an opposing leg opening edge, comprising the steps of:
- interposing and affixing the absorbent core between the topsheet and the backsheet to assemble an absorbent chassis which comprises the topsheet, the backsheet and the absorbent core therebetween, and which has a pair of sides and a longitudinal centerline dividing the chassis into halves wherein the absorbent chassis is divided into a crotch region, a front waist region and a back waist region and the front waist region and the back waist region extend oppositely from the crotch region along the longitudinal centerline;
- putting the leg opening edges of the pre-stretched elastic panels on each side of the chassis;
- bonding the elastic panels together with the topsheet and the backsheet along the side edge of the chassis to integrate the elastic panels and the chassis;
- folding the elastic panels in such a manner that the waist opening edge of the elastic panel extends at least along the longitudinal centerline of the chassis,
- attaching the elastic panels to the chassis along attachment lines which comprise a front attachment line connecting a point near the longitudinal centerline in the front waist region of the chassis to a point on each side of the chassis and a back attachment line connecting a point near the longitudinal centerline in the back waist region of the chassis to a point on each side of the chassis whereby the waist opening edges of the elastic panels form completely the waist opening of the diaper; and
- forming leg openings in regions along each leg opening edge of the elastic panels.

According to the fourth aspect of the present invention we provide a method for preparing a disposable pull-on diaper having a waist opening and leg openings, comprising the steps of:
- providing an absorbent core, a covering sheet and a single sheet which comprises a chassis area and side areas extending from each side of the chassis area, wherein each side area has a side edge, with the proviso that when the single sheet is liquid-pervious, the covering sheet is liquid-impervious, and vice versa;
- interposing and affixing the core between the chassis area of the single sheet and the covering sheet, to assemble a chassis portion which comprises the chassis area of the single sheet, the covering sheet and the core therebetween, and which has a longitudinal centerline dividing the chassis portion into halves wherein the absorbent chassis portion is divided into a crotch region, a front waist region and a back waist region, and the front waist region and the back waist region extend oppositely from the crotch region along the longitudinal centerline;
- elasticizing the side areas of the single sheet to form elastic panels having a side edge;
- forming leg openings along a borderline between the chassis area and each elastic panel;
- folding elastic panel along the borderline in such a manner that the side edge of the elastic panels extend to at least the longitudinal centerline of the chassis;
- attaching the elastic panels to the chassis portion along attachment lines which comprises a front attachment line connecting a point near the longitudinal centerline in the front waist region of the chassis portion to a point on the borderline and a back attachment line connecting a point near the longitudinal centerline in the back waist region of the chassis portion to a point on the borderline, whereby the side edges of the elastic panels form completely the waist opening of the diaper.

### BRIEF DESCRIPTION OF TEE DRAWINGS

While the specification concludes with claims particularly pointing out and distinctly claiming the present invention, it is believed that the same will be better understood from the following description taken in conjunction with the accompanying drawings in which:
Figure 1 is a perspective view showing one embodiment of a disposable pull-on diaper according to the present invention in the configuration it would assume when placed on a wearer;
Figure 2A is a perspective view of the diaper in Figure 1 in its flat state;
Figure 2B is a partially cutway view showing the diaper in Figure 2A;
Figure 2C is a cross-sectional view taken along section line B-B of the Figure 2A;
Figure 2D is a cross-sectional view taken along section line C-C of the Figure 2A;
Figure 2E is a cross-sectional view taken along section line C-C of an alternative embodiment of the diaper of Figure 2A;
Figure 3A is a perspective view showing another embodiment of a disposable pull-on diaper according to the present invention in its flat state;
Figure 3B is a perspective view showing another embodiment of a disposable pull-on diaper according to the present invention in its flat state;
Figure 3C is a perspective view showing another embodiment of a disposable pull-on diaper according to the present invention in its flat state;
Figure 4A is a perspective view showing another embodiment of a disposable pull-on diaper according to the present invention in its flat state;
Figure 4B is a perspective view showing diaper in Figure 4A in the configuration it would assume when placed on a wearer;
Figure 5A is a bottom view of a diaper having pleats on a chassis in its flat state;
Figure 5B is a bottom view of a diaper having a pleats on a chassis in the configuration it would assume when placed on a wearer;
Figure 5C is a side view of a diaper having pleats on elastic side panels;
Figure 6A is a perspective view showing another embodiment of a disposable pull-on diaper according to the present invention in its flat state;
Figure 6B is a cross-sectional view taken along section line D-D of the Figure 6A;
Figures 6C, 6D and 6E show other preferred embodiments of the present invention;
Figure 7A illustrates an assembly of a diaper according to the present invention;
Figure 7B illustrates a final assembly of a diaper according to the present invention;
Figure 7C illustrates diagrammatically a process for a continuous manufacture of a diaper according to the present invention;
Figure 7D is a cross-sectional view illustrating an assembly of the diaper in Figure 6A;
Figure 7E illustrates a preferred process for preparing the diaper in Figure 6A;
Figure 7F is a cross-sectional view illustrating an assembly of the diaper in Figure 6A;
Figure 7G illustrates another preferred process for preparing the diaper in Figure 6A;
Figure 8A is a perspective view showing a diaper having a disposal securing means;
Figure 8B illustrates a soiled diaper which is rolled up and secured with the disposal securing means;
Figure 8C is an enlarged cross-sectional view of a z-folded adhesive tape tab which is preferably used as the disposal securing means in Figure 8A.

### DETAILED DESCRIPTION OF THE INVENTION

Referring to the drawings, there is shown a preferred embodiment of the present invention as it would be used in a disposable diaper intended to be worn by an infant.

As used herein, the term "disposable pull-on pants-style diaper" refers to a garment generally worn by infants or incontinent persons, which is pulled on like pants, and which is intended to be discarded after a single use (i.e., it is not intended to be laundered or otherwise restored and reused).

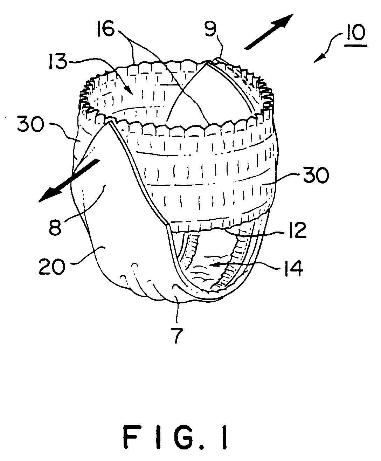

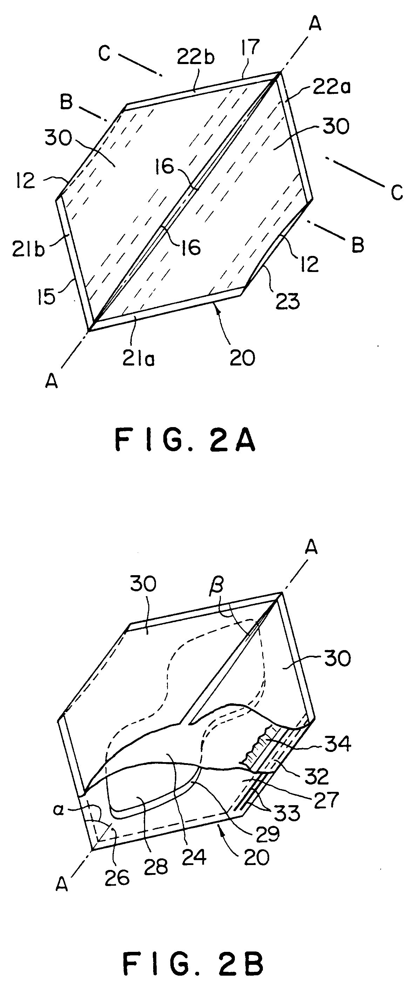

Figure 1 is a perspective view of the diaper 10 of the present invention in the configuration it would assume when placed on a wearer. As can be seen in Figure 1, a preferred diaper 10 basically comprises an absorbent chassis 20 and elastic panels 30 and has a waist opening 13 and a pair of leg openings 14. The chassis 20 comprises three regions, a crotch region 7, a front waist region 8 and a back waist region 9. The front waist region and the back waist region extends oppositely from the crotch region along a longitudinal direction of the chassis 20. When the diaper 10 is pulled downward towards the front and the back (indicated by arrows in Figure 1), the diaper is able to take a flat state shown in Figure 2A.

Figure 2A is, thus, a perspective view of the diaper 10 of the present invention in its flat state. As shown in Figure 2A, the absorbent chassis 20 has the front waist region 8 having a front edge 15, the back waist region 9 having a back edge 17, and a pair of sides 23, and the elastic panels 30 are attached to the wearer-facing surface of the absorbent chassis 20 along front attachment line 21 on the front edge 15 and along back attachment line 22 on the back edge 17. Each elastic panel 30 has a waist opening edge 16 and a leg opening edge 12.

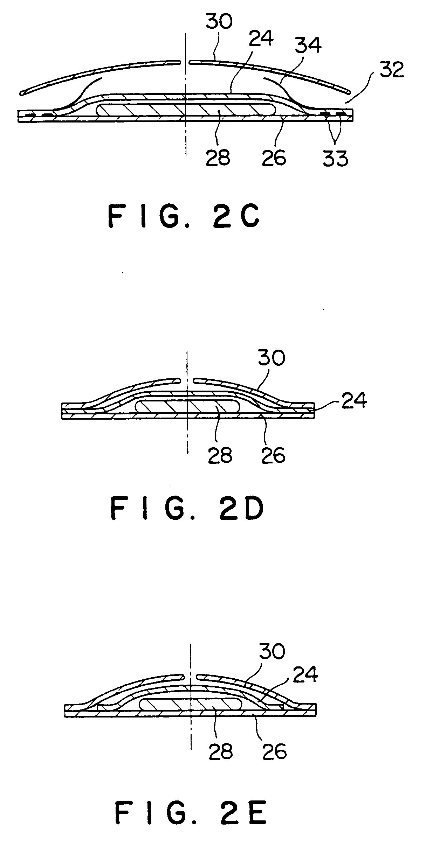

Figure 2B is a perspective view of the diaper with portions of the structure being cut-away to more clearly show the construction of the diaper 10. Furthermore, Figure 2C is a cross-sectional view of the diaper 10 taken along section line B-B of Figure 2A. The diaper 10 preferably comprises the absorbent chassis 20 comprising a liquid-pervious topsheet 24; a liquid-impervious backsheet 26; an absorbent core 28 interposed between the topsheet 24 and the backsheet 26; and elastic panels 30. The diaper 10 can optionally, though preferably, comprise elasticized flaps 32 and barrier leg cuffs 34. A topsheet side of the chassis 20 forms an inner or wearer-facing surface, and a backsheet side of the chassis 20 forms an outer or garment-facing surface. That is, the inner surface comprises that portion of the diaper 10 which is facing toward the wearer's body during use, and the outer surface comprises that portion of the diaper 10 which is facing toward the wearer's garments or clothing. The chassis 20 is shown in Figure 2B to have a longitudinal centerline A-A which divides the chassis into halves, preferably symmetrical halves.

Figure 2B shows a preferred embodiment of the chassis 20 in which the topsheet 24 and the backsheet 26 have length and width dimensions generally larger than those of the absorbent core 28. The topsheet 24 and the backsheet 26 extend beyond the edges of the absorbent core 28 to thereby form an area 27 between the side edge 23 and the side edge 29 of the core. While the topsheet 24, the backsheet 26, and the absorbent core 28 can be assembled in a variety of well known configurations, preferred chassis configuration are described generally in U.S. Patent 4,636,207, issued to Buell on Jan. 13, 1987; U.S. Patent 4,610,678, issued to Weisman et al. on Sept. 9, 1986; U.S. Patent 4,695,278, issued to Lawson on Sept. 22, 1987.

As illustrated in Figure 2A, each elastic panel 30 is attached to the chassis 20 along a portion of the front edge 15 represented by front attachment line 21 and along a portion of the back edge 17 of the chassis represented by the back attachment line 22. The front attachment line 21a, 21b each is a line which connects, preferably symmetrically connects, a point near the longitudinal centerline, preferably on the centerline, on the front edge 21 of the chassis to a point on each respective side of the chassis, and the back attachment line 22a, 22b each is a line which connects, preferably symmetrically connects, a point near the longitudinal centerline, preferably on the centerline, on the back edge of the chassis to a point on each respective side of the chassis. As shown in Figure 2B, attachment lines 21a, 21b and 22a, 22b are substantially straight lines. However, they can also be concave or convex curvilinear relative to a center of the chassis 20. The attachment lines 21 and 22 form with the side edges 23 a substantially hexagonal area on the topsheet side of the chassis. An angle α formed by a front attachment line 21a, 21b with the longitudinal centerline A-A can be the same as or different from an angle β formed by a back attachment line 22a, 22b with the longitudinal centerline A-A. Preferably, angles α and β, independently, are from 20°-60°, more preferably about 45°. "Substantially hexagonal" means that the attachment lines 21 and 22 can be slightly curved and provide an area having a generally hexagonal shape.

Furthermore, each elastic panel 30 is provided on the topsheet side to cover a respective half of the chassis, and the waist opening edges 16 are registered with the longitudinal centerline of the chassis. In a preferred embodiment of the present invention, each waist opening edge 16 extends to the longitudinal centerline of the chassis. The elastic panels 30 which extend at least to the longitudinal centerline of the chassis will form completely the waist opening 13. The waist opening 13 which is fully elasticised will be adapted to contact directly with a whole waist of the wearer. This ensures an improved fit of the diaper on the waist of wearer.

As described, the elastic panels 30 are attached to the chassis 20 along the attachment lines 21 and 22. The elastic panel 30 can be attached to either the topsheet 24 or other structure situated on the garment-facing surface of the chassis or directly to the garment-facing surface of the backsheet 28, as long as the elastic panels 30 are provided on the topsheet side of the chassis 20. Figures 2D and 2E are cross-sectional views of of the diaper 10 taken along section line C-C of Figure 2A. The preferred embodiment as shown in Figure 2D has a topsheet which fully registers with the backsheet 26. In the embodiment, the elastic panels 30 are attached on the topsheet 24. Another preferred embodiment as shown in Figure 2E, on the other hand, shows a chassis which has a wearer-fading surface which is partly formed by a wearer-facing surface of the backsheet 26. This is because the topsheet 24 does not fully cover the periphery of the backsheet 26. In the embodiment, elastic panels 30 are attached to a wearer-facing surface along at least a portion of the periphery of the backsheet 26.

The elastic panels 30 in the flat state shown in Figures 2A and 2B are stretched. Hence, when the force which makes the diaper 10 take the flat state is once released, the elastic means provided in the elastic panel is relaxed. The effective length of the relaxed panel becomes short and the pant is formed. It is preferable that the elastic panel which is relaxed has a length along the waist opening edge 16 of about 20 to 30% the longitudinal length of the chassis 20 along the centerline A-A. In other words, the relaxed waist opening 12 preferably has a circumference which is about 20 to 30% twice the longitudinal length of the chassis 20 at centerline A-A.

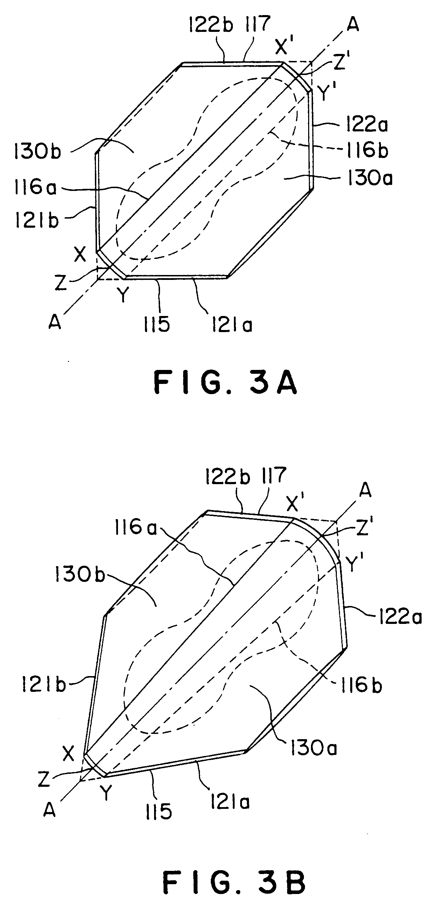

In another preferred embodiment of the present invention shown in Figure 3A, the pair of elastic panels 130 have a waist opening edge 116 which extends beyond the centerline A-A of the chassis. Each elastic panel 130, in the flat state, covers not only its respective half of the chassis, but extends also to cover a portion of the opposing half of the chassis. In this embodiment, the respective elastic panels 130a and 130b overlap, one over the other, in an area defined by their respective waist opening edges 116a and 116b, front attachment lines 121a, 121b and back attachment lines 122a, 122b. The benefit of this construction is a substantial reduction in the circumference of the waist opening of the diaper, which can improve the fit of the diaper around the waist opening. As shown in Figure 3A, the circumference of the waist opening 116 in the flat state is the sum of the waist opening edges 116a and 116b, less the distance that the elastic panels 130a, 130b overlap at the front of the chassis (the distance between points X-Y), and less the distance that the elastic panels overlap at the rear of the chassis (the distance between points X'-Y').

The amount of overlap of the respective elastic panels effects also the fit and appearance of the diaper as worn. In this embodiment, the elastic panels 130a and 130b are attached to the chassis along the front attachment line 121 on the front edge 115 of the chassis and the back attachment line 122 on the back edge 117 of the chassis. The front attachment line 121a, 121b each is a line which connects, preferably symmetrically connects, a point designated as Z in Figure 3A to a point on each side of the chassis, and the back attachment line 122a, 122b each connects, preferably symmetrically connects, a point designated as Z' in Figure 3A to a point on each side of the chassis. As shown in the figure, the points Z and Z' are near, and preferably on, the longitudinal centerline A-A. In Figure 3A, the attachment lines are shown to be bent at points X, Y, X' and Y' so that the attachment lines and the longitudinal centerline cross at nearly right angles. It is within the scope of the present invention that attachment lines need not be straight lines, and that the attachment lines form with the side of the chassis a substantially hexagonal area on the wearer-facing surface. In addition, in this embodiment the lines X-Y and X'-Y' which are portions of the attachment lines are slightly curved and intersect with lines X-X' and Y-Y'. However different line patterns, such as straight lines and intersecting outboard the lines X-X' and Y-Y' are within the ordinary skill of the artisan. In order to improve the appearance and fit of the diaper, the resulting redundant corner portions outside of the lines X-Y and X'-Y' can be cut away.

It is also within the scope of the present invention that the two overlapped elastic panels 130 have respective waist opening edges 116a, 116b which are not parallel to each other. As shown in Figure 3B, the front portions of the overlapping elastic panels are extended to and attached along the front attachment lines 121a, 121b at points closer to the centerline A-A than the respective attachment points of the rear portions of the elastic panels along attachment lines 122a, 122b. In such case, the direction of elastic stretch and retraction in the portion of the elastic panel closest to the waist opening edge is preferably parallel to the respective waist opening edge 116, rather than to the longitudinal centerline of the chassis.

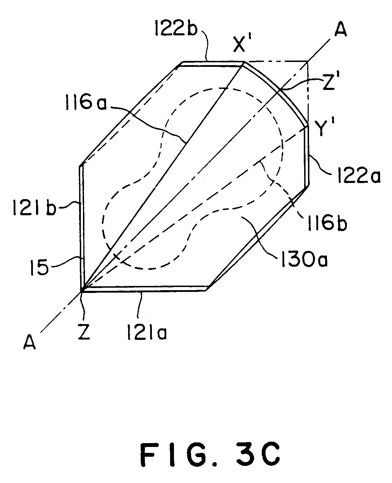

Another related embodiment having the elastic panels 130a, 130b having overlapping waist opening edges 116a, 116b is shown in Figure 3C. Front portions of the elastic panels extend to and meet at the front edge 15 of the chassis near the centerline A-A (that is, at point Z), whereas the rear portions of the elastic panels each extend across the centerline and overlap in the area defined by lines Z-X', Z-Y' and X'-Y'. As previously described, the rear portion of the chassis can be sealed along line X'-Y', and the redundant rear corner removed to improve the appearance and fit of the diaper.

The embodiments shown in Figures 3B and 3C can reduce the circumference of the waist opening, and can provide different front and back shape in the diaper to take into account the anatomy of the wearer.

The absorbent core 28 can be any absorbent means which is generally compressible, conformable, non-irritating to the wearer's skin, and capable of absorbing and retaining liquids such as urine and other certain body exudates. The absorbent core has a garment surface, a body surface, and side edges. The absorbent core 28 can be manufactured in a wide variety of sizes and shape (e.g., rectangular, hourglass, dog-bone-shaped, "T"-shaped, asymmetric, etc.) and can form a wide variety of liquid absorbent material commonly used in disposable diaper and other absorbent articles such as comminuted wood pulp which is generally referred to as airfelt. Examples of other suitable absorbent materials include creped cellulose wadding; meltblown polymers including coform; chemically stiffened, modified or cross-linked cellulosic fibers; tissue including tissue wraps and tissue laminates; absorbent foams; absorbent sponges; superabsorbent polymers; absorbent gelling materials; or any equivalent material or combinations of materials. The configuration and construction of the absorbent core can also be varied (e.g., the absorbent core can have varying caliper zones, a surface pattern of embodiments or compressions in any direction to improve lateral and longitudinal distribution of fluid, a hydrophilic gradient, a superabsorbent gradient, or lower average density and lower average basis weight acquisition zones; or can comprise one or more layers or structures). The total absorbent capacity of the absorbent core 28 should, however, be compatible with the design loading and the intended use of the diaper 10. Further, the size and absorbent capacity of the absorbent core 28 can be varied to accommodate wearers ranging from infants through to adults. Exemplary absorbent structures for use as the absorbent core 28 are described in U.S. Patent 4,610,678 entitled "High-Density Absorbent Structures" issued to Weisman et al. on September 9, 1986; U.S. Patent 4,673,402 entitled "Absorbent Articles With Dual-Layered Cores" issued to Weisman et al. on June 16, 1987; U.S. Patent 4,888,231 entitled "Absorbent Core Having A Dusting Layer" issued to Angstadt on December 19, 1989; and U.S. Patent 4,834,735 entitled "High-Density Absorbent Members Having Lower Density and Lower Basis Weight Acquisition Zones" issued to Alemamy et al. on May 30, 1989.

The backsheet 26 is impervious to liquids (e.g., urine) and is preferably manufactured from a thin plastic film, although other flexible liquid impervious materials can also be used. As used herein, the term "flexible" refers to materials which are compliant and will readily conform to the general shape and contours of the human body. The backsheet prevents the exudates absorbed and contained in the absorbent core 28 from wetting articles which contact the diaper 10 such as undergarments and bedsheets. The backsheet 26 can thus comprise a woven or nonwoven material, polymeric films such as thermoplastic films of polyethylene or polypropylene, or composite materials such as a film-coated nonwoven material. Preferably, the backsheet is a thermoplastic film having a thickness of from about 0.012 mm (0.5 mil) to about 0.051 mm (2.0 mils). The backsheet can be embossed to provide a matte finish to the plastic film which can improve tactility and appearance. Particularly preferred materials for the backsheet include polyethylene films, such as one manufactured by Monsanto Chemical Corporation and designated Film No. 8020.

The backsheet 26 is positioned adjacent the garment surface of the absorbent core and is preferably joined thereto by attachment means such as those well known in the art. For example, the backsheet can be secured to the absorbent core by a uniform continuous layer of adhesive, a patterned layer of adhesive, or an any of separate lines, spirals, or spots of adhesive. Adhesive which have been found to be satisfactory are manufactured by Century Adhesive, Inc. of Columbus, Ohio and marketed as Century 5227; and by H.B. Fuller Company of St. Paul, Minnesota and marketed as HL-1258. The attachment means will preferably comprise an open pattern network of filaments of adhesive as is disclosed in U.S. Patent 4,573,986 entitled "Disposable Waste-Containment Garment", which issued to Minetola et al. on March 4, 1986, more preferably several lines of adhesive filaments swirled into a spiral pattern such as is illustrated by the apparatus and methods shown in U.S. Patent 3,911,173 issued to Spraque, Jr. on October 7, 1975; U.S. Patent 4,785,996 issued to Ziecker et al. on November 22, 1978; and U.S. Patent 4,842,666 issued to Werenicz on June 27, 1989. Alternatively, the attachment means can comprise heat bonds, pressure bonds, ultrasonic bonds, dynamic mechanical bonds, or any other suitable attachment means or combinations of these attachment means as are known in the art.

Optionally, an outer backing sheet can be registered with or attached to the garment-facing surface of the backsheet to provide an improvement in the appearance and the skin feel of the garmet-facing surface the disposable diaper. Materials for such outer backing sheets can include, for example skin-friendly materials such as those described herein after for the topsheet, as well as apertured or embossed formed films.

The topsheet 24 is positioned adjacent the body surface of the absorbent core 28 and is preferably affixed thereto and to a portion of the backsheet 26 by attachment means (not shown) such as those well known in the art. Suitable attachment means are described with respect to joining the backsheet 26 to the absorbent core 28. As used herein, the term "affixed" encompasses configurations whereby an element is directly secured to the other element by affixing the element directly to the other element, and configurations whereby the element is indirectly secured to the other element by affixing the element to intermediate member(s) which in turn are affixed to the other element. In a preferred embodiment of the present invention, the topsheet 24 and the backsheet 26 are affixed directly to each other in the area between the side edge 23 and the side edge 29 of the core 28 and are indirectly joined together by directly joining them to the respective sides of the absorbent core 28 by the attachment means (not shown).

The topsheet 24 is compliant, provides a soft feeling, and is non-irritating to the wearer's skin. Further, the topsheet 24 is liquid pervious, permitting liquids (e.g., urine) to readily penetrate through its thickness, and thereby isolating the wearer's skin from the absorbent core containing the liquids. A suitable topsheet can be manufactured from a wide range of materials, such as porous foams; reticulated forms; apertured plastic films; or woven or nonwoven webs of natural fibers (e.g., wood or cotton fibers), synthetic fibers (e.g., polyester or polypropylene fibers), or a combination of natural and synthetic fibers. There are a number of manufacturing techniques which can be used to manufacture the topsheet 24. For example, the topsheet 24 can be a nonwoven web of fibers spunbonded, carded, wet-laid, meltblown, hydroentangled, combinations of the above, or the like. A preferred topsheet is one manufactured by Hercules, Inc. as Type 151 polypropylene fibers which comprises staple length polypropylene fibers having a denier of about 1.5, and fiber length of at least about 15.9 mm (0.625 inches); or, one manufactured by Fiberweb North America and available as 80/20 polypropylene/rayon carded thermally bonded nonwoven.

The elastic panel 30 is a sheet or a panel which comprises an elastic means and which can be stretched and, upon relaxing, will tend to resume essentially its original shape. Elastic panel 30 can be made as disclosed in U.S. Patents US-A-4,940,464; 4,938,753; and 4,938,757 all of which issued to Van Gompel et al., U.S. Patent 4,107,364 issued to Sisson on June 24, 1980, U.S. Patent 4,525,407 issued to Ness on June 25, 1985, U.S. Patent 4,834,741 issued to Sabee on May 30, 1989, European Patent Publication 409,315, The Procter & Gamble Company, published January 23, 1991. Elastic panels can also be made as described in United States Patents US-A-5,167,897, US-A-5,156,793, and US-A-5,143,679, each filed on February 28, 1991 and Japanese Patent Application No. 155637/1992 filed on May 22, 1992. A preferred elastic panel 30 can be formed, for example, by attaching a pre-stretched elastic strand, string, or film as an elastic means between a pair of nonwovens, or by laminating a shearable nonwoven to a stretchable film (as an elastic means) and "activating" it as described in the above Japanese Patent Application. (As used herein, a "prestretched, elastic strand, string or film" can include a heat shrinkable elastomer film). In brief, by "activating" is meant that the laminated web is mechanically stretched incrementally so that alternating sections of the nonwoven are purposely deformed or torn. Thus, when the "activated" laminate is pulled, it will stretch (unimpeded by nonwoven) in the direction of activation.

Suitable elastic strings, strands, or films can be made from materials including: synthetic or natural rubber, such as LYCRA, elastomeric scrim, elastomeric films (including heat shrinkable elastomeric films), elastomeric woven or nonwoven webs, elastomeric composites such as elastomeric nonwoven laminates, synthetic or natural rubber foams, and the like. A preferred elastomeric foam for use as the elastic means can include: cross-linked natural rubber foams preferably having a caliper of approximately 35 mils and a density of 13 pounds per cubic foot (0.214 grams per cubic centimeter), such as is available from Fulflex Inc. of Middletown, Rhode Island; or as available from Ludlow Composites Corporation of Fremont, Ohio; or polyurethane foams having a caliper of about 80 mils and a density of about 2 pounds per cubic foot (0.033 grams per cubic centimeter) such as is available from Bridgestone of Yokohama, Japan and marketed under the tradename Bridgestone SG polyurethane foam.

Suitable nonwoven materials for use in making the elastic panels can be selected from those identified herein before for use as the topsheet material.

Preferably, the elastic panels of the present invention are elastically extensible in at least the longitudinal (viewed in the flat state) direction. The term "elastically extensible" means that when tensional forces are applied, the elastic panel will return to about its previous size and shape after the tensional force is removed. An elastic panel of the present invention will preferably return to at least 75% of their original configuration within about 5 seconds or less upon stretch and immediate release thereof (i.e., a "snappy" elastic). More preferably, the elastic panel will return to at least 95%-98% of their original configuration after such stretch and release. Furthermore, an elastic panel preferably will stretch by about 200% to 500%, more preferably from about 300% to about 500%, from its relaxed state to its fully stretched state such as in the flat view. To provide this stretchability, a preferred elastic means for the waist portion and the leg opening portion of the elastic panel is an elastic strand made from natural rubber having a diameter about 0.5-4.0 mm, more preferably about 2.5 mm. For the elastic means in the portion of the elastic panel between the waist portion and the leg opening portions, a preferred elastic strand is made of Opelon having a diameter of about 0.2-1.0 mm, preferably about 0.5 mm and is supplied by DuPont-Toray Co., Ltd. By selecting an elastic means having the appropriate extension modules, stretch, and retraction force, the elastic panels can provide the diaper with good body conformity and comfort, without excessive sagging or slip which can result in poor fit and poor containment.

To improve the "snappy" elastic characteristic of the elastic panel that has elastic strands or strings as the elastic means, it is preferred to secure the elastic strands to the nonwoven layer or layers of the elastic panel at their terminal ends only, or at least at a limited number of attachment points along the length of the elastic strand.

Ordinarily, adhesive is applied along the length of a pre-stretched elastic strand to affix it to the nonwoven. When secured, the tensional force is released from the elastic strand and it tends to shrink in length to its uninhibited, relaxed length. However, as the elastic strands contract and the elastic panel gathers, the nonwoven attachment along the length of the elastic strand can exert an opposite force to the contraction force of the elastic. This can result in the elastic panel having a "relaxed" length that is considerably longer than the uninhibited, relaxed length of the individual strand. This can result in a substantially larger waist opening than is desired or expected, and reduced fit and comfort.

Preferably, the elastic strands are attached only at their ends. The two (or more) layers of nonwoven with the elastic strand in between are joined by a seal along a seal portion that is positioned on each side along the length of the elastic strand. The seal along the seal portion can be continuous or intermittent. As a result, the elastic strand is encased within a pocket bounded by the two nonwoven layers and the side portions on each side of the elastic. The seal can be made by mechanical means or by an adhesive, as described herein before.

Preferably, portions of the nonwovens along the seal portion can be intermittently removed to reduce the stiffness, and hence the resistance, caused by the sealing of the nonwoven itself.

Alternatively, portions of the elastic strand along its length can be affixed, preferably with an adhesive, to one or both nonwoven layers to help hold the elastic strand in place. The adhesive can be applied to the elastic strand by conventional means, such as by spiral or droplet spray patterns. Suitable adhesives and adhesive application methods are those described herein above for securing the backsheet to the absorbent core.

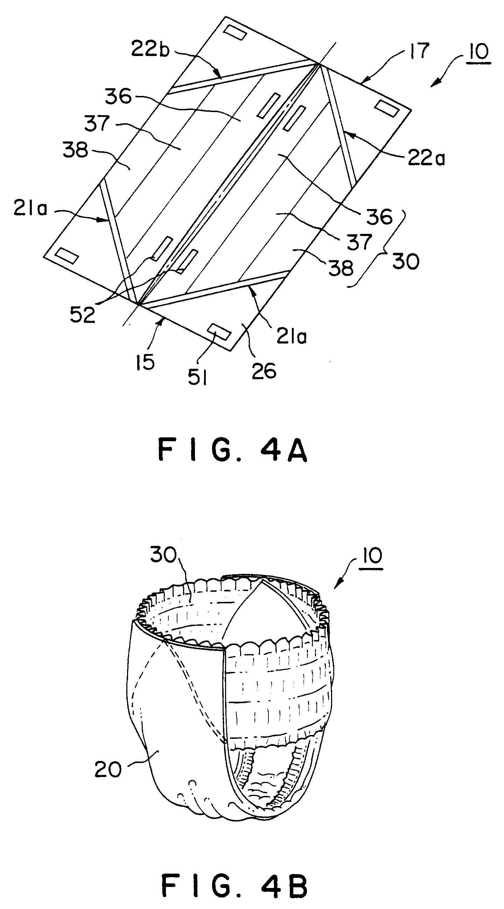

An alternative embodiment of diaper 10 is shown in Figures 4A and 4B, where the diaper 10 is illustrated as including a chassis 20 which is rectangular. As shown in Figure 4A, the elastic panels 30 are attached to the chassis along the attachment lines 21, 22 as described above. In this embodiment of the diaper, the front attachment line 21a, 21b comprises a line connecting, preferably symmetrically connecting, a point near the longitudinal centerline, preferably on the centerline on the front edge 21 to a point on each side of the chassis 20, the back attachment line 22a, 22b comprises a line connecting, preferably symmetrically connecting, a point near the longitudinal centerline, preferably on the centerline on the back edge 22 to a point on each side of the chassis 20. In this embodiment, the front edge 15 of the chassis 20 and the front attachment line 21 do not coincide, and the back edge 17 of the chassis and the back attachment line 22 do not coincide. The elastic panels 30 are then attached along the attachment lines 21, 22. In this embodiment, the redundant corners, defined by the front edge 15 (or back edge 17), the chassis side edge 23, and the front attachment line 21 (or back attachment line 22) of the chassis, are not removed. In order to prevent the corners of the chassis from flapping, the diaper preferably has a fastening means for attaching a corner of the chassis to the elastic panel 30. It should be understood that the use of a material which can engage elements should be non-limiting in the sense that the engaging elements can comprise any shape as are known in the art so long as they are adapted to engage elements. A preferred fastening means is a fastener disclosed in Japanese Patent Application No. 21897/1990, filed on January 31, 1990. The diaper 10 shown in Figure 4A has fasteners 51 and 52 on the corner of the chassis and the elastic panel 30, respectively. Figure 4B shows the diaper 10 which is formed. As shown in Figure 4B, the corners of the chassis are fastened to the elastic panels 30.

An added advantage of such hooks is to provide the ability of the user to adjust the waist opening fit of the diaper by cinching or shortening the elastic panels and thereby shortening the waist circumference of the diaper.

A preferred embodiment as described herein can further comprise elastic panels as shown in Figure 4A, and described hereinafter. Each panel has a plurality of regions along its width which can have different retraction forces. In Figure 4A, the three regions of the elastic panel 30, i.e., the regions designated as 36, 37 and 38, have different retraction force. The region 36 typically has the greatest retraction force among the three regions, and the region 38 has a greater force than the region 37. The elastic panel 30 which comprises the regions having different retraction force provides an advantage in that the greatest retraction force is provided in those areas most required, such as the waist opening and the leg opening regions. An elastic panel which has regions having different retraction force can preferably be made by attaching on a single sheet, or between two sheets, elastic strands which have been pre-stretched to different degree, or have different retraction or elastic properties. Different type, size, thickness, and number of elastic means can be employed to provide the different retraction forces or elastic properties.

An elastic panel 30 having regions having different retraction force can also be made by combining discrete members having the different retraction force along their edges into a unitary panel 30. The individual panel members can be made from different elastic means (that is, for example, having different elastic strand or stretchable film types), or from the same elastic means (such as elastic strands) and pre-stretched more or less prior to attachment with the other panel members.

In a preferred embodiment as shown in Figure 2B, the diaper 10 can have leg elastic flaps 32. The elastic flaps 32 can be made by any means well known in the art. For example, the elastic flaps 32 can be made by inserting and attaching pre-stretched elastic strands 33 between the topsheet 24 and the backsheet 26 along the side edges 23 of the chassis. The elastic flaps 32 form, with the leg opening edge 23 of the elastic panel, an elasticized leg opening 14. The elasticized leg openings provide an improved fit and better comfort. Furthermore, the leg elastic flaps 32 can provide an improved containment of liquid and other body exudates.

The diaper 10 preferably further comprises barrier leg cuffs 34 for providing improved containment of liquid and other body exudates. Each leg cuff 34 can comprise several different embodiments for reducing the leakage of body exudates in the leg regions. While this type of leg cuff is sometimes also referred to as leg bands, side flaps, barrier cuffs, or elastic cuffs, the meaning of these words is intended to be the same. U.S. Patent 3,860,003 describes a disposable diaper which provides a contractible leg opening, a side flap and one or more elastic members to provide an elasticized leg cuff (gasketing cuff). U.S. Patent 4,909,803 entitled "Disposable Absorbent Article Having Elasticized Flaps" issued to Aziz et al. on March 20, 1990, describes a disposable diaper having "stand-up" elasticized flaps (barrier cuffs) to improve the containment of the leg regions. U.S. Patent 4,695,278 entitled "Absorbent Article Having Dual Cuffs" issued to Lawson on September 22, 1987, describes a disposable diaper having dual cuffs including a gasketing cuff and a barrier cuff.

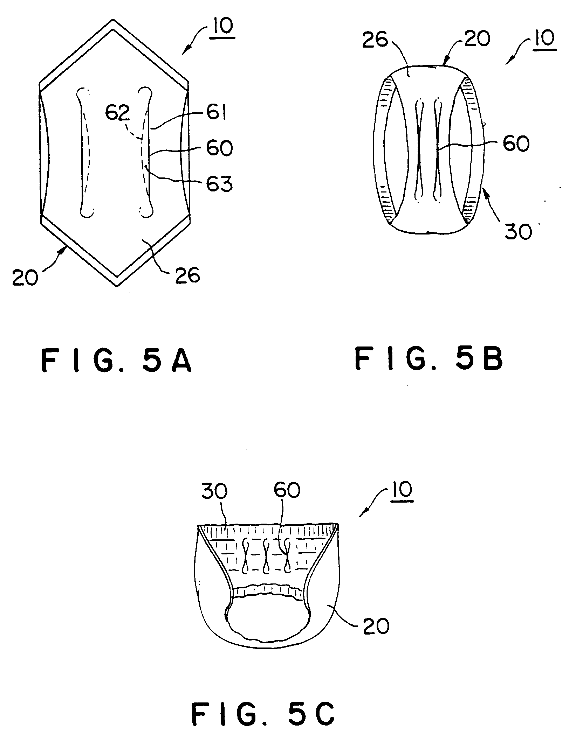

The diaper 10 preferably further comprises one or more pleats on a chassis 20 or elastic panels 30. A diaper having pleats on a chassis is illustrated in Figures 5A and 5B. Figure 5A is a bottom view of the diaper 10 which takes a flat state, and Figure 5B is a bottom view of the diaper 10 which is formed. As shown in the figures, the backsheet 26 is pleated so that a crotch portion of the chassis is narrowed by pulling the crotch portion of the chassis toward the center. Preferably the pleating of the crotch area is made more toward the front or the diaper to better conform the leg openings to the anatomy of the wearer. The narrowed crotch portion can provide less restricted movement of legs as well as better comfort and fit preferably. Generally a plurality of pleats are employed. The pleating can be made in the backsheet only; or in the backsheet and topsheet together in the area 27 between the edge 29 of the absorbent core and the edge 23 of the chassis; or in the absorbent core, topsheet and backsheet of the chassis area itself. Each pleat has two fold lines 61 and 62. The backsheet surface 63 between the two fold lines 61 and 62 is folded over the backsheet material outside of the second fold line 62, and is secured in this position by a pleat restraint (not shown) which can be continuous or intermittent, at the center of the pleat or along its length, and can be a mechanical sealing or an adhesive sealing of the backsheet portion 63 inside of the pleat to the backsheet material outside of the second fold line 62. The pleats can be linear or curvilinear, and can range in lengths from 20 mm to 200 mm, depending on the number of pleats and the positioning of the pleats. Preferably, a plural of pleats are placed side-by-side.

Figure 5C is a side view of a diaper 10 having pleats on the elastic panels 30, oriented in the lateral direction, perpendicular to the direction of stretch of the elastic panel. The pleats on the elastic panels 30 provide a slightly smaller waist opening and leg opening without shortening the elastic panel 30 itself. Alternatively, the pleats can be made in the elastic panel oriented in the longitudinal direction, parallel to the direction of stretch of the elastic panel. Such pleats can provide a larger and more rounded leg opening, thus improving the fit and comfort of the diaper.

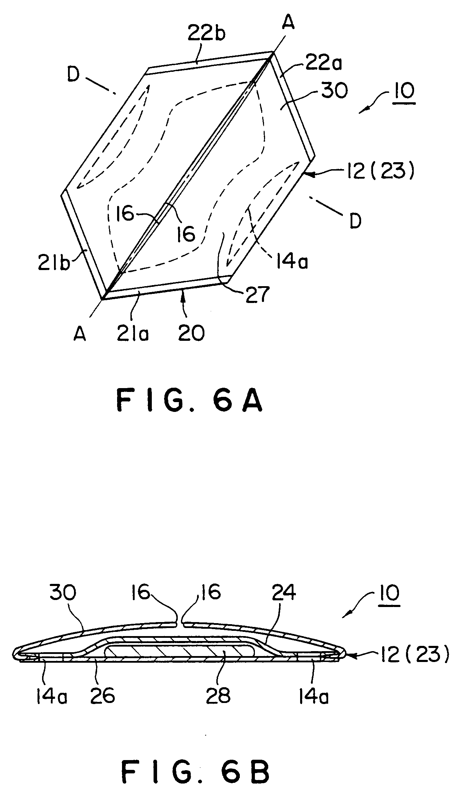

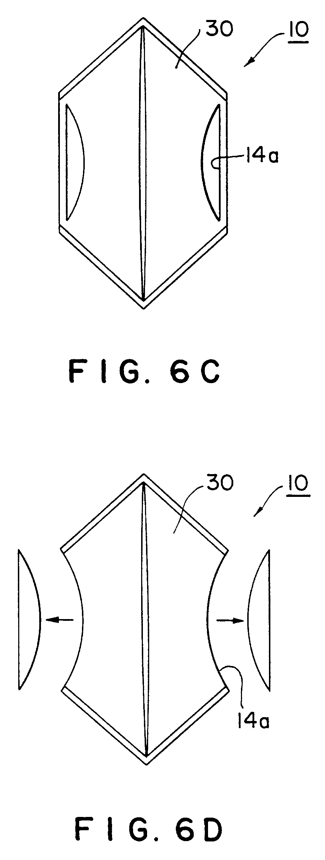

Figures 6A and 6B show another preferred embodiment of the present invention in which leg opening edges 12 of the elastic panels 30 are connected to sides 23 of the chassis 20, and leg openings 14a are formed through the chassis 20 in an area between a side edge 23 of the chassis 20 and a side edge 29 of an absorbent core 28.

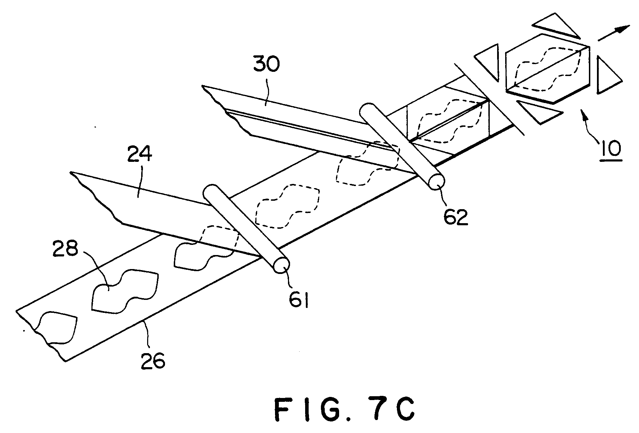

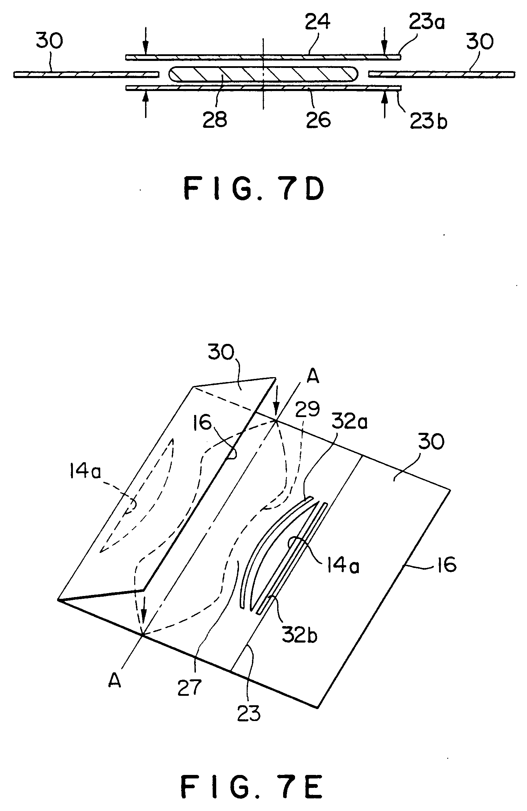

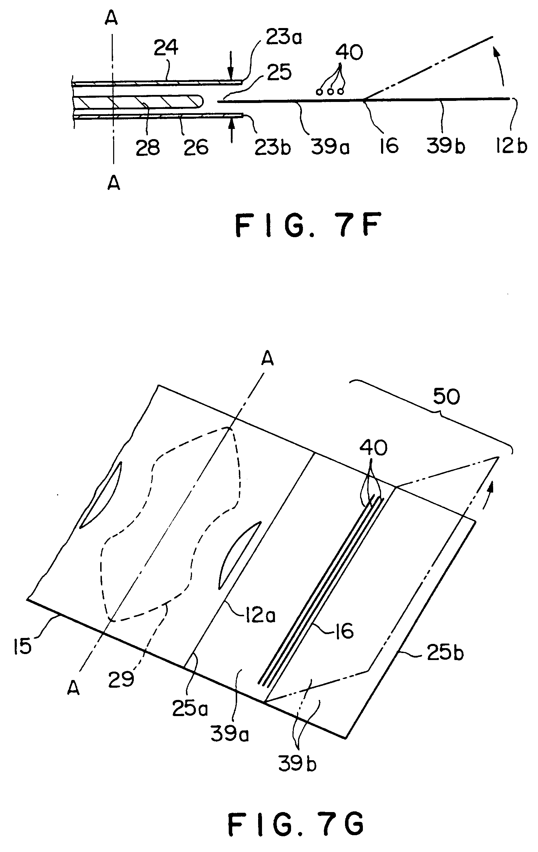

Figure 6C shows another preferred embodiment similar to the embodiment of Figure 6A, except that the leg openings are formed through the elastic panels themselves, instead of through the chassis. The leg opening 14a in the elastic panel can be made prior to attachment of the elastic panels to the chassis. In the method of attaching the elastic panels shown in Figures 7C, 7E, 7F and 7G, the leg openings can also be formed in the elastic panel after it is attached to the side edge of the chassis, but preferably before the elastic panel is folded over and attached to the topsheet-surface of the chassis. In this embodiment, the elastic means can be positioned on either or both sides of the leg opening in order to provide leg elastics therefore.

As shown in Figure 6D, another preferred embodiment of the present invention provides that leg openings can be formed by removing (for example by cutting or stamping) portions of the chassis and the elastic panel along the side of the chassis. Alternatively, portions of either the chassis, or of the elastic panel, alone can be removed, whereby an improved leg opening and leg opening fit can be achieved. Preferably, the leg opening edges of the elastic panel and the chassis are provided with elastic flaps 32 and barrier cuffs 34 to improve the fit and exudate containment of the diaper.

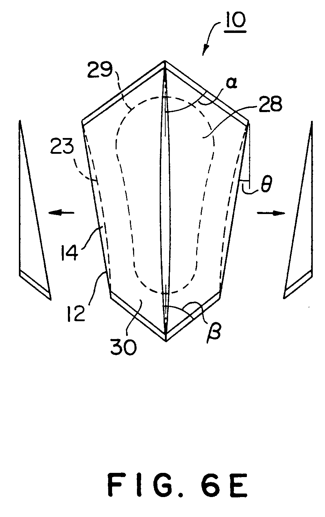

A particularly preferred embodiment of the present invention is shown in Figure 6E. The resultant diaper chassis of this embodiment is formed or cut so that the front portion and the back portion of the chassis are asymmetric with respect to a lateral centerline through the chassis. In Figure 6E, the opposing side edges 23 of the chassis and the opposing leg opening edges 12 of the elastic panels are not parallel with each other or with the longitudinal centerline of the chassis. By tapering inward (by an angle θ) the leg opening edge and the edge of the chassis from the back to the front of the diaper, an improved fit and appearance can be achieved for the leg opening and the diaper in general. A wider core and chassis area in the back and narrower core and chassis in the front provide less leg obstruction in the front, for better fit for children and adults. The angle θ of the taper can be selected up to about 30°, more preferably from about 5°-25°. The selection of the angle of θ also will depend on the selection of the angles α and β of the attachment lines 21 and 22, and the desired fit and appearance. Alternatively in this embodiment, the side edge 23 of the chassis and the leg opening edge 12 of the elastic panel can be joined and the leg opening formed in chassis (as shown in Figure 6A as 14a) or in the elastic panel itself (as shown in Figure 6C as 14a). Preferably, the elastic means provided in the elastic panel in the region of the leg opening 14 is oriented parallel to the leg opening edge 12, and the elastic means of the elastic flaps 32 attached to the chassis are oriented parallel to the edge 23 of the chassis.

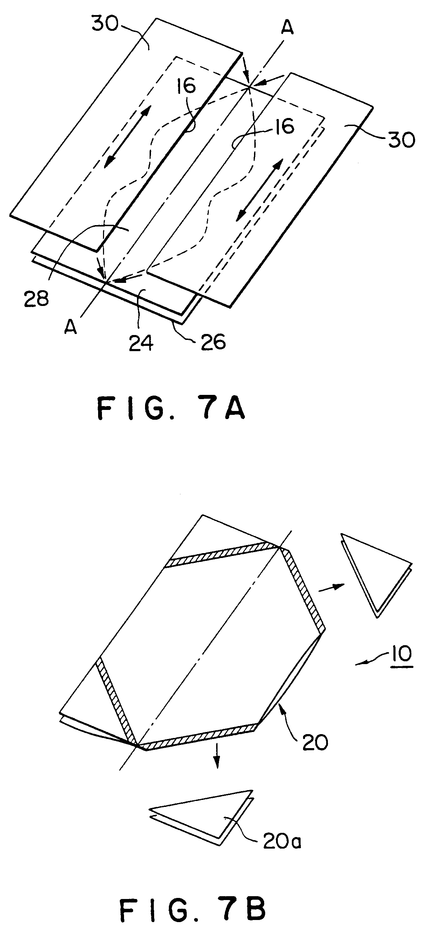

According to another aspect of the present invention there is provided a method for preparing the diaper 10 as shown in Figure 1. The method will be explained referring to Figures 7A, 7B and 7C. As shown in Figure 7A, the absorbent core 28 can be first interposed between the topsheet 24 and the backsheet 26 and then the chassis 20 can be assembled according to the preferred manner as described above with respect to assembling the chassis 20. The topsheet 24 and the backsheet 26 can be affixed with the absorbent core prior to attachment of the elastic panels, or can be affixed by a means which at the same time affixes the elastic panels 30 to the chassis 20, as described below. The elastic panels 30 are stretched in the longitudinal direction as indicated by arrows in Figure 7A, and then attached to each half of the chassis 20 with the waist opening edge 16 registered with the longitudinal centerline of the chassis 20, i.e., the line A-A in Figure 7A. The waist opening edge 16 can optionally be extended beyond the longitudinal centerline on either or both the front and the back ends of the chassis, as herein before described. The pre-stretched panels 30 are attached to the chassis 20 along the attachment lines 21 and 22. The attachment of the pre-stretched panels to the chassis can be made by treating the attachment line 21 and 22 areas with mechanical energy sufficient to fuse the elastic panel material to the chassis material. As used herein, "mechanical energy" includes also thermal energy such as that employed in a heat seal. Such mechanical energy can be applied preferably in the form of an ultrasonic apparatus or pinch roller, which are well known for such purposes. A preferred mechanical seal is a pressure bond seal which bonds together the thermoplastic material of the elastic panels to the thermoplastic material of the chassis, whether to the topsheet or to the wearer-facing surface of the backsheet. In one suitable method, the chassis assembly having the pre-stretched elastic panel superposed thereon is passed between a rotating anvil roll and a rotating pressure roll which typically exert about 1500-5000 kilograms force per square centimeter pressure on elastic panel-chassis assembly, thereby forming an attachment line having a width generally from 1-10 mm, preferably from 2-5 mm. The bonding pattern can be continuous or intermittent, and straight, curved, or irregular. Preferably, a temperature below about 80 degrees centigrade is used. It is most preferred to operate the anvil and the pressure roll at their ambient temperature. A higher temperature can be used so long as it is well below the thermoplastic melting temperature of the materials of the elastic panel or the chassis, to avoid damaging or weakening these materials at such temperatures.

Another preferred method is a heat seal made by heating the rotating anvil roll and/or the rotating pressure roll at or above the melting temperature of the thermoplastic materials of the elastic panels and chassis, and applying a considerably lower pressure to avoid excessive damage and weakening of the nonwoven fabric layers in the area of the attachment lines.

The attachment can also be made by using an adhesive or chemical seal to bond the materials of the elastic panel and chassis together. A suitable adhesive for such purpose is a double-sided adhesive tape from Minesota Mining and Manufacturing Co. (3M), designated as code #1524. The resultant diaper 10 in a flat view is illustrated in Figure 7B. As shown in Figure 7B, the corners 20a of the chassis 20 can be optionally removed to obtain a substantially hexagonal chassis in the flat view.

Figure 7C is a schema of one apparatus for conducting the method described above. In the apparatus, a strip of successively adjoining backsheets 26 are supplied in a longitudinal direction by a supply means (not shown). A supply roll 61 operates to guide a strip of successively adjoining topsheets 24 onto the strip of the backsheets 26 on which absorbent cores 28 are preliminary provided at longitudinally spaced positions by a supply means (not shown), so that the absorbent cores 28 are interposed between the strip of topsheets 24 and the strip of backsheets 28 thereby forming an absorbent chassis 20. After this, onto the strip of topsheets 24 are supplied by means of a supply roll 62, two parallel strips of a continuous web of elastic panels 30, or one roll of a web which is split into two strips along its centerline, which are pre-stretched prior to application to the chassis. At a position downstream of the supply roll 62 the resultant assembly of the strips is attached, i.e., by heat seals, or adhesive, to the chassis. The assembled diaper is then cut off transversely into the individual diapers by cutting means (not shown). The redundant corners of the diaper can be optionally removed outside the elastic attachment lines to obtain a substantially hexagonal shape of diaper 10 in the flat view.

The diaper 10 as shown in Figure 6A can preferably be made by the following method which forms the third aspect of the present invention. As illustrated in Figure 7D, elastic panels 30 are placed with an edge 25 thereof registered with each side edge 23 of the chassis 20. Preferably the edge 25 of the elastic panel 30 is positioned between the respective edges 23a, 23b of the topsheet 24 and the backsheet 26. The resultant assembly is then bonded, e.g., by mechanical seals or adhesive as herein before described, along edges 23 at positions indicated by arrows in Figure 7D. The leg openings 14a can be formed through the chassis 20 by cutting or stamping prior to, or after, the step of bonding the resultant assembly described above.

As shown in Figure 7E, embodiments provided herein which have a leg opening 14a in the area 27 of the chassis between the side edge 23 and the edge 29 of the absorbent can have one or more leg elastic flaps 32a, 32b positioned around its periphery to provide improved fit, comfort, and containment in the leg opening.

The elastic panels 30 are then folded in such a manner that the waist opening edges 16 are extended at least to the longitudinal centerline A-A of the chassis 20 as shown in Figure 7E. In this embodiment, the waist opening edge 16 can also be extended beyond the longitudinal centerline, as previously described. Thereafter, the elastic panels 30 are attached to the chassis 20 along the attachment lines 21 and 22, and removal of the corners of the chassis 20 and elastic panels 30 gives the diaper 10 as shown in Figure 6A.

According to a preferred embodiment of the present invention, the elastic panel 30 as shown in Figures 7D and 7E can be made from a panel 50 having double width of the elastic panel 30. As illustrated in Figures 7F and 7G, the pre-stretched elastic means 40, preferably elastic strands, are provided on, preferably attached to, the double-width panel 50 along the line 16 which divides the double-width panel 50 into portions 39a and 39b. After the panel 50 is folded along the line 16, the portion 39b can be attached, such as by adhesive or mechanical seal, to the portion 39a, thereby forming the elastic panel 30. It is preferable that the inboard edge 25a of the panel 50 is registered with the outboard edge 25b, and the edges 25a and 25b are positioned between the respective edges 23a and 23b of the topsheet 24 and the backsheet 26 before the assembly.

According to the fourth aspect of the present invention, in order to eliminate the step of bonding the resultant assembly described above, a single sheet can be used to form both one of the layers of the absorbent chassis 20, such as the backsheet 28 or the topsheet 24, and the elastic panels 30. Thus, the single sheet has a chassis area and side areas, extending from each side of the chassis area, and the side areas are provided with elastic means. The side areas can be preferably elasticized by a manner previously described herein before, for elasticizing an elastic panel 30. After or before the elasticizing step of the side areas, the absorbent core 28 is interposed between the chassis area of the single sheet and a covering sheet to assemble a chassis portion which comprises the chassis area of the single sheet, the covering sheet and the core therebetween. When the single sheet is liquid-pervious and can perform as a topsheet, the covering sheet should be liquid-impervious and perform as a backsheet. Alternatively, when the single sheet is liquid-impervious and can perform as a backsheet, the covering sheet should be liquid-pervious and perform as a topsheet. From the resultant assembly, the diaper can be made by the steps described as the method of the third aspect of the present invention.

From the methods described above for forming elastic panels and attaching them to the chassis, the various embodiments described herein before can be made. Methods of attaching leg elastics 32 and barrier cuffs 34 are well known as herein before mentioned. In these embodiments where the elastic means is oriented and affixed to the chassis or the elastic panels in a direction non-parallel with the longitudinal centerline (in the direction of manufacture), known means exist for positioning the attachment of the elastic any lateral distance from the centerline as the chassis or elastic panels move along a manufacturing apparatus, such as that apparatus shown in Figure 7C. For example, in the construction of leg elastics 32 for the embodiment shown in Figure 6E, an elastic means (such as elastic strands) can be applied to and attached to the backsheet (outside the edge 29 of absorbent core 24) by a guide means (not shown) which is reciprocated in the lateral direction (perpendicular to movement of the chassis), in order to adjust the position of attachment of the elastic strand in the lateral direction, as the each chassis passes by the guide means. The cycle of reciprocation is adjusted and timed to coincide with the speed of the chassis elements that pass by the guide. By adjusting the speed of a guide as it reciprocates through a cycle, straight, diagonal, or curvilinear lines of elastic strands can be formed onto the chassis. Alternatively, elastic strands could be attached to the topsheet, prior to attachment of the topsheet to the chassis. By a similar method, elastic means can also be attached into the elastic panels to provide elastic means oriented in a non-longitudinal direction.

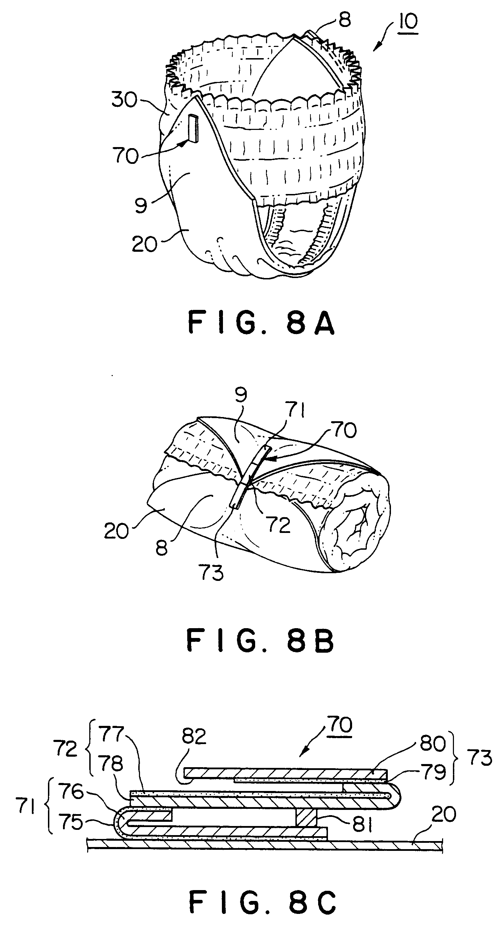

As shown in Figure 8A, a preferred pull-on diaper can also comprise a disposal securing means 70 for securing a soiled diaper after it has been removed from the wearer before disposal. Conventional diapers ordinarily have a pair of adhesive tape or mechanical tabs on the side edges of the back waist portion of the conventional diaper, and suitable landing areas on the front waist portion, for securing the conventional diaper to the wearer. In the case of a pull-on diaper, there is ordinarily no need for such tape tabs to secure the diaper around the waist of the wearer, this purpose instead being served by the elastic panels.

In the case of pull-on diaper of the present invention, the soiled diaper is preferably removed by separating the elastic panel 30 from the chassis 20 along the front attachment lines 21. The soiled diaper can then be rolled up from the front waist area 8 to the back waist area 9 and secured with the disposal securing means 70 as shown in Figure 8B. Preferably, the disposal securing means 70 is oriented in the longitudinal direction and is attached to the outer or garment-facing surface of the chassis 20 in the back waist area 9 to facilitate this disposal method.

Though any conventional adhesive tape or mechanical tape tab can be used the disposal securing means 70, a preferred disposal securing means is a three-section, z-folded adhesive tape tab as shown in Figure 8C. Adhesive tape tab 70 comprises a first section 71, a second section 72, and a third section 73. First section 71 is secured by pressure-sensitive adhesive means 75 to the chassis 20, and has one end secured by said same pressure-sensitive adhesive means 75 to one end of second section 72. Second section 72 is attached at its other end to third section 73 with pressure-sensitive adhesive means 79 which is well known in the art. Second section 72 can have a releasing means 77 on the surface facing third section 73 which provides the pressure-sensitive adhesive means 79 with moderate adhesion thereto. A suitable releasing means 77 can be a coating of silicon or a release liner made of polyethylene. Releasing means 77 can also be a low-adhesion pressure-sensitive adhesive on the surface facing third section 73 which is compatible with pressure-sensitive adhesive means 79, which means that the low-adhesion pressure-sensitive adhesive means 77 will adhere to, but can be separated from, adhesive means 79 without significantly effecting the adhesive quality of the respective adhesive means 77 and 79. Prior to use in securing the diaper, second section 72 is separably attached to first section 71 by a lower-adhesion coating 81.

To use the z-folded tape tab 70, the user extends the tape tab by pulling on the portion 82 of third section 73, which causes the third section 73 to separate from second section 72, which causes second section 72 to unfold at fold 78 and to separate from first section 71, and which causes first section 71 to unfold at fold 76. The adhesive means 75, 77, and 79 of first section 71, second section 72, and third section 73, respectively, then can be used to secure the extended tape tab 70 to the outer, garment-facing surface of the rolled-up diaper chassis 20.

A similar adhesive tape tab is disclosed in German Patent DE-A-4033850 filed October 24, 1990 (3M Company, Minneapolis, MN).

Having shown and described the preferred embodiment of the present invention, further adaptions of the disposable diaper described herein can be accomplished by appropriate modifications by one of ordinary skill in the art without departing from the scope of the present invention. Accordingly, the scope of the present invention should be considered in terms of the following claims and is understood not to be limited to the details of structure and operation shown and described in the specification and drawings.

## Claims

1. A disposable pull-on diaper (10) having a waist opening (13) and leg openings (14), Comprising, in a flat state:
an absorbent chassis (20) Comprising a liquid pervious topsheet (24), a liquid impervious backsheet (26) and an absorbent core (28) having a pair of side edges interposed between the topsheet (24) and the backsheet 26 , the chassis (20) having a pair of sides (23), a front edge (15), a back edge (17), and a longitudinal centreline A-A which divides the chassis (20) into halves, the chassis (20) being divided into a crotch region (7), a front waist region (8) having the front edge (15) and a back waist region (9) having the back edge (17) wherein the front waist region (8) and the back waist region (9) extend oppositely from the crotch region (7) along the longitudinal centreline A-A;
at least one elastic panel (30) attached to the wearer-facing surface of each half of the chassis (20), the waist opening edge (16) of each elastic panel (30) extending at least to the longitudinal centreline A-A of the chassis (20), each elastic panel (30) being attached to the chassis (20) along attachment lines (21, 22),
a leg opening (14) in the region along the leg opening edge (12) of the elastic panel (30);
characterised in that
each elastic panel (30) having a waist opening edge (16) and an opposing leg opening edge (12), attachment lines (21, 22) which comprise a front attachment line (21) connecting a point near the longitudinal centreline A-A in the front waist region (8) of the chassis (20) to a point on each side of the chassis (20) and a back attachment line (22) connecting a point near the longitudinal centreline A-A in the back waist region (9) of the chassis (20) to a point on each side of the chassis (20); and
wherein the elastic panels (30) form completely the waist opening (13).

2. A diaper (10) according to claim 1, wherein the leg opening edge (12) of the elastic panel (30) is registered with the side of the chassis (20), and each leg opening (14) is defined by the side of the chassis (20) and the leg opening edge (12) of the elastic panel (30).

3. A diaper (10) according to claim 1, wherein each elastic panel (30) is connected along the leg opening edge (12) thereof to the respective side (23) of the chassis (20), and each leg opening (14) is formed through the chassis (20) in an area between the side edge (23) of the chassis (20) and the side edge (29) of the core (28).

4. A diaper (10) according to claim 1, wherein each elastic panel (30) is connected along the leg opening edge (12) thereof to the respective side (23) of the chassis (20), and each leg opening (14) is formed through the elastic panel (30).

5. A diaper (10) according to claim 1, wherein the point from which the front attachment line (21) starts is on the front edge (15) of the chassis (20), and the point from which the back attachment line (22) starts is on the back edge (17) of the chassis (20).

6. A diaper (10) according to claim 1, wherein the points from which the front attachment line (21) and the back attachment line (22) start respectively are on the longitudinal centerline (A-A) the chassis (20).

7. A diaper (10) according to claim 1, wherein the chassis (20) has front and back edges (15, 17) which are defined by the respective front and back attachment lines (21, 22).

8. A diaper (10) according to claim 1, wherein pleats are made near each leg opening (14) by securing one or more folds made in a portion of the chassis (20).

9. A diaper (10) according to claim 1, wherein the elastic panels (30) have regions having different retractive forces along width thereof.

10. A diaper (10) according to claim 1, wherein each elastic panel (30) extends beyond the longitudinal centerline (A-A) to cover a portion of the opposing half of the chassis (20), whereby one elastic panel (30) covers a portion of the other elastic panel (30), thereby forming a diaper (10) having a reduced waist opening (13).

11. A diaper (10) according to clam 1, wherein the leg opening edge (12) of the elastic panel (30) and the side edge (23) of the chassis (20) are tapered inward from the back to the front of the diaper (10), thereby forming a diaper (10) with a wider chassis (20) in the back than in the front.

12. A method for preparing a disposable pull-on diaper (10) having a waist opening (13) and leg openings (14) having a liquid-pervious topsheet (24), a liquid-impervious backsheet (26) an absorbent core (28) and a pair of elastic panels (30) each having a waist opening edge (16) and an opposing leg opening edge (12), comprising the steps of:
- interposing and affixing the absorbent core (28) between the topsheet (24) and the backsheet (26) to assemble an absorbent chassis (20) which has a pair of sides (23) and a longitudinal centerline (A-A) dividing the chassis (20) into halves and which comprises the topsheet (24), the backsheet (26) and the absorbent core (28) interposed therebetween wherein the absorbent chassis (20) is divided into a crotch region (7), a front waist region (8) and a back waist region (9) and the front waist region (8) and the back waist region (9) extend oppositely from the crotch region (7) along the longitudinal centerline (A-A);
characterised in that said steps further comprise
- registering a pre-stretched elastic panel (30) with each half of the chassis (20) in such a manner that the waist opening edge (16) of the elastic panel (30) extends at least along the longitudinal centerline (A-A) of the chassis (20); and
- attaching the pre-stretched elastic panel (30) to the chassis (20) along attachment lines (21, 22) which comprise a front attachment line (21) connecting a point near the longitudinal centerline (A-A) in the front waist region (8) of the chassis (20) to a point on each side of the chassis (20) and a back attachment line (22) connecting a point near the longitudinal centreline A-A in the back waist region (9) of the chassis (20) to a point on each side of the chassis (20) whereby the waist opening edges (16) of the elastic panels (30) form completely the waist opening (13) of the diaper (10).

13. A method for preparing a disposable pull-on diaper (10) according to claim 12
characterised in that said steps further comprise
- putting the leg opening edges (12) of the prestretched elastic panels (30) on each side of the chassis (20);
- bonding the elastic panels (30) together with the topsheet (24) and the backsheet (26) along the side edge (23) of the chassis (20) to integrate the elastic panels (30) and the chassis (20);
- forming leg openings (14) in regions along each leg opening edge (12) of the elastic panels (30).

14. A method for preparing a disposable pull-on diaper (10) according to claim 12
characterised in that said steps further comprise
- elasticising the side areas of the topsheet (24) to form elastic panels (30) having a side edge (25);
- forming leg openings (14) along a borderline between the chassis (20) and each elastic panel (30);
- folding elastic panel (30) along the borderline in such a manner that the side edge (25) of the elastic panels extend to at least the longitudinal centreline (A-A) of the chassis (20).

15. A method for preparing a diaper (10) according to claims 12, 13 or 14, wherein the elastic panel (30) is folded in such a manner that the waist opening edges (16) extend beyond the longitudinal centreline A-A of the chassis (20) to cover a portion of the opposing half of the chassis (20), whereby one elastic panel (30) covers a portion of the other elastic panel (30).

## Patentansprüche

1. Eine wegwerfbare Anzieh-Windel (10) mit einer Taillenöffnung (13) und Beinöffnungen (14), welche, in einem flachen Zustand, umfaßt:
eine absorbierende Grundstruktur (20), welche ein flüssigkeitsdurchlässiges Deckblatt (24), ein flüssigkeitsundurchlässiges Rückenblatt (26) und einen absorbierenden Kern (28) mit einem Paar Seitenrändern, welche zwischen dem Deckblatt (24) und dem Rückenblatt (26) dazwischengelegt sind, umfaßt, wobei die Grundstruktur (20) ein Paar Seiten (23), einen vorderen Rand (15), einen hinteren Rand (17) und eine Längsmittellinie A-A, welche die Grundstruktur (20) in Hälften teilt, aufweist, die Grundstruktur (20) in einen Schrittbereich (7), einen den vorderen Rand (15) enthaltenden vorderen Taillenbereich (8) und einen den hinteren Rand (17) enthaltenden hinteren Taillenbereich (9) unterteilt ist, wobei der vordere Taillenbereich (8) und der hintere Taillenbereich (9) sich entlang der Längsmittellinie A-A entgegengesetzt vom Schrittbereich (7) erstrecken;
mindestens ein elastisches Feld (30), welches mit der dem Träger zugewandten Oberfläche jeder Hälfte der Grundstruktur (20) verbunden ist, wobei der Taillenöffnungsrand (16) jedes elastischen Feldes (30) sich mindestens über die Längsmittellinie A-A der Grundstruktur (20) erstreckt, jedes elastische Feld (30) mit der Grundstruktur (20) entlang Befestigungslinien (21, 22) verbunden ist,
eine Beinöffnung (14) im Bereich entlang dem Beinöffnungsrand (12) des elastischen Feldes (30);
dadurch gekennzeichnet, daß
jedes elastische Feld (30) einen Taillenöffnungsrand (16) und einen gegenüberliegenden Beinöffnungsrand (12), Befestigungslinien (21, 22), welche eine vordere Befestigungslinie (21), welche einen Punkt nahe der Längsmittellinie A-A im vorderen Taillenbereich (8) der Grundstruktur (20) mit einem Punkt an jeder Seite der Grundstruktur (20) verbindet, und eine hintere Befestigungslinie (22), welche einen Punkt nahe der Längsmittellinie A-A im hinteren Taillenbereich (9) der Grundstruktur (20) mit einem Punkt an jeder Seite der Grundstruktur (20) verbindet, umfassen, aufweist;
und bei welcher die elastischen Felder (30) vollständig die Taillenöffnung (13) bilden.

2. Eine Windel (10) nach Anspruch 1, bei welcher der Beinöffnungsrand (12) des elastischen Feldes (30) mit der Seite der Grundstruktur (20) übereingestimmt ist und jede Beinöffnung (14) von der Seite der Grundstruktur (20) und dem Beinöffnungsrand (12) des elastischen Feldes (30) begrenzt ist.

3. Eine Windel (10) nach Anspruch 1, bei welcher jedes elastische Feld (30) entlang seinem Beinöffnungsrand (12) mit der jeweiligen Seite (23) der Grundstruktur (20) verbunden ist und jede Beinöffnung (14) durch die Grundstruktur (20) in einer Zone zwischen dem Seitenrand (23) der Grundstruktur (20) und dem Seitenrand (29) des Kerns (28) gebildet ist.

4. Eine Windel (10) nach Anspruch 1, bei welcher jedes elastische Feld (30) entlang seinem Beinöffnungsrand (12) mit der jeweiligen Seite (23) der Grundstruktur (20) verbunden ist und jede Beinöffnung (14) durch das elastische Feld (30) gebildet ist.

5. Eine Windel (10) nach Anspruch 1, bei welcher der Punkt,von welchem die vordere Befestigungslinie (21) ausgeht, sich am vorderen Rand (15) der Grundstruktur (20) befindet und der Punkt, von welchem die hintere Befestigungslinie (22) ausgeht, sich am hinteren Rand (17) der Grundstruktur (20) befindet.

6. Eine Windel (10) nach Anspruch 1, bei welcher die Punkte, von welchen die vordere Befestigungslinie (21) und die hintere Befestigungslinie (22) ausgehen, sich jeweils an der Längsmittellinie A-A der Grundstruktur (20) befinden.

7. Eine Windel (10) nach Anspruch 1, bei welcher die Grundstruktur (20) vordere und hintere Ränder (15, 17) aufweist, welche von den jeweiligen vorderen und hinteren Befestigungslinien (21, 22) definiert sind.

8. Eine Windel (10) nach Anspruch 1, bei welcher Falten nahe jeder Beinöffnung (14) durch Fixieren einer oder mehrerer Falten, welche in einem Abschnitt der Grundstruktur (20) hergestellt sind, erzeugt sind.

9. Eine Windel (10) nach Anspruch 1, bei welcher die elastischen Felder (30) Bereiche aufweisen, welche entlang deren Breite unterschiedliche Retraktionskräfte aufweisen.

10. Eine Windel (10) nach Anspruch 1, bei welcher jedes elastische Feld (30) sich über die Längsmittellinie A-A hinaus erstreckt, um einen Abschnitt der gegenüberliegenden Hälfte der Grundstruktur (20) zu bedecken, wodurch ein elastisches Feld (30) einen Abschnitt des anderen elastischen Feldes (30) bedeckt, wodurch eine Windel (10) mit einer reduzierten Taillenöffnung (13) gebildet wird.

11. Eine Windel (10) nach Anspruch 1, bei welcher der Beinöffnungsrand (12) des elastischen Feldes (30) und der Seitenrand (23) der Grundstruktur (20) vom Rückenteil zum Vorderteil der Windel einwärts spitz zulaufen, wodurch eine Windel (10) mit einer breiteren Grundstruktur (20) im Rückenteil als im Vorderteil gebildet wird.

12. Ein Verfahren zum Herstellen einer wegwerfbaren Anzieh-Windel (10) mit einer Taillenöffnung (13) und Beinöffnungen (14), welche ein flüssigkeitsdurchlässiges Deckblatt (24), ein flüssigkeitsundurchlässiges Rückenblatt (26), einen absorbierenden Kern (28) und ein Paar elastische Felder (30), wobei jedes einen Taillenöffnungsrand (16) und einen gegenüberliegenden Beinöffnungsrand (12) aufweist, umfaßt, welches folgende Schritte umfaßt:
- Dazwischenlegen und Fixieren des absorbierenden Kerns (28) zwischen das Deckblatt (24) und das Rückenblatt (26), um eine absorbierende Grundstruktur (20) zusammenzusetzen, welche ein Paar Seiten (23) und eine Längsmittellinie A-A, welche die Grundstruktur (20) in Hälften teilt, umfaßt, und welche das Deckblatt (24), das Rückenblatt (26) und den absorbierenden Kern (28) dazwischengelegt aufweist, wobei die absorbierende Grundstruktur (20) in einen Schrittbereich (7), einen vorderen Taillenbereich (8) und einen hinteren Taillenbereich (9) unterteilt ist und der vordere Taillenbereich (8) und der hintere Taillenbereich (9) sich vom Schrittbereich (7) entlang der Längsmittellinie A-A entgegengesetzt erstrecken; dadurch gekennzeichnet, daß die genannten Schritte weiters umfassen:
- Abstimmen eines vorgestreckten elastischen Feldes (30) mit jeder Hälfte der Grundstruktur (20) auf eine derartige Weise, daß der Taillenöffnungsrand (16) des elastischen Feldes (30) sich mindestens entlang der Längsmittellinie A-A der Grundstruktur (20) erstreckt;
- Fixieren des vorgestreckten elastischen Feldes (30) an der Grundstruktur (20) entlang Befestigungslinien (21, 22), welche eine vordere Befestigungslinie (21), welche einen Punkt nahe der Längsmittellinie A-A im vorderen Taillenbereich (8) der Grundstruktur (20) mit einem Punkt an jeder Seite der Grundstruktur (20) verbindet, und eine hintere Befestigungslinie (22), welche einen Punkt nahe der Längsmittellinie A-A im hinteren Taillenbereich (9) der Grundstruktur (20) mit einem Punkt an jeder Seite der Grundstruktur (20) verbindet, umfassen, wodurch die Taillenöffnungsränder (16) der elastischen Felder (30) vollständig die Taillenöffnung (13) der Windel (10) bilden.

13. Ein Verfahren zum Herstellen einer wegwerfbaren Anzieh-Windel (10) gemäß Anspruch 12,
dadurch gekennzeichnet, daß die Schritte weiters umfassen:
- Anordnen der Beinöffnungsränder (12) der vorgestreckten elastischen Felder (30) an jeder Seite der Grundstruktur (20);
- Verbinden der elastischen Felder (30) zusammen mit dem Deckblatt (24) und dem Rückenblatt (26) entlang dem Seitenrand (23) der Grundstruktur (20), um die elastischen Felder (30) und die Grundstruktur (20) zu vereinigen;
- Bilden von Beinöffnungen (14) in Bereichen entlang jedem Beinöffnungsrand (12) der elastischen Felder (30).

14. Ein Verfahren zum Herstellen einer wegwerfbaren Anzieh-Windel (10) gemäß Anspruch 12,
dadurch gekennzeichnet, daß die genannten Schritte weiters umfassen:
- Elastischmachen der Seitenzonen des Deckblatts (24), um elastische Felder (30) mit einem Seitenrand (25) zu bilden;
- Bilden von Beinöffnungen (14) entlang einer Begrenzungslinie zwischen der Grundstruktur (20) und jedem elastischen Feld (30);
- Falten des elastischen Feldes (30) entlang der Begrenzungslinie auf eine derartige Weise, daß der Seitenrand (25) der elastischen Felder sich mindestens zur Längsmittellinie A-A der Grundstruktur (20) erstreckt.

15. Ein Verfahren zum Herstellen einer Windel (10) nach den Ansprüchen 12, 13 oder 14, bei welchem das elastische Feld (30) auf eine derartige Weise gefaltet wird, daß die Taillenöffnungsränder (16) sich über die Längsmittellinie A-A der Grundstruktur (20) hinaus erstrecken, um einen Abschnitt der gegenüberliegenden Hälfte der Struktur (20) zu bedecken, wodurch ein elastisches Feld (30) einen Abschnitt des anderen elastischen Feldes (30) bedeckt.

## Revendications

1. Couche à enfiler à jeter après usage (10) ayant une ouverture de ceinture (13) et des ouvertures de jambe (14), comprenant, à l'état plat :
- une structure absorbante (20) comportant une feuille de dessus perméable aux liquides (24), une feuille de fond imperméable aux liquides (26) et une âme absorbante (28) ayant une paire de bords latéraux interposés entre la feuille de dessus (24) et la feuille de fond (26), la structure (20) ayant une paire de côtés (23), un bord avant (15), un bord arrière (17), et une ligne médiane longitudinale A-A qui divise la structure (20) en deux moitiés, la structure (20) étant divisée en une partie d'entrejambe (7), une partie de ceinture avant (8) ayant le bord avant (15) et une partie de ceinture arrière (9) ayant le bord arrière (17) dans laquelle la partie de ceinture avant (8) et la partie de ceinture arrière (9) s'étendent à l'opposé à partir de la partie d'entrejambe (7) le long de la ligne médiane longitudinale A-A ;
- au moins un panneau élastique (30) fixé à la surface tournée vers l'utilisateur de chaque moitié de la structure (20), le bord de l'ouverture de ceinture (16) de chaque panneau élastique (30) s'étendant au moins jusqu'à la ligne médiane longitudinale A-A de la structure (20), chaque panneau élastique (30) étant fixé à la structure (20) le long de lignes de fixation (21, 22),
- une ouverture de jambe (14) dans la partie située le long du bord de l'ouverture de jambe (12) du panneau élastique (30) ;
caractérisée en ce que
chaque panneau élastique (30) comporte un bord d'ouverture de ceinture (16) et un bord d'ouverture de jambe opposé (12), des lignes de fixation (21, 22) qui comportent une ligne de fixation avant (21) reliant un point, à proximité de la ligne médiane longitudinale A-A dans la partie de ceinture avant (8) de la structure (20), à un point sur chaque côté de la structure (20) et une ligne de fixation arrière (22) reliant un point à proximité de la ligne médiane longitudinale A-A dans la partie de ceinture arrière (9) de la structure (20), à un point sur chaque côté de la structure (20) ; et
dans laquelle les panneaux élastiques (30) forment complètement l'ouverture de ceinture (13).

2. Couche (10) selon la revendication 1, dans laquelle le bord d'ouverture de jambe (12) du panneau élastique (30) coïncide avec le côté de la structure (20) et chaque ouverture de jambe (14) est délimitée par le côté de la structure (20) et le bord d'ouverture de jambe (12) du panneau élastique (30).

3. Couche (10) selon la revendication 1, dans laquelle chaque panneau élastique (30) est relié le long de son bord d'ouverture de jambe (12) au côté respectif (23) de la structure (20) et chaque ouverture de jambe (14) est formée à travers la structure (20) dans une surface entre le bord latéral (23) de la structure (20) et le bord latéral (29) de l'âme (28).

4. Couche (10) selon la revendication 1, dans laquelle chaque panneau élastique (30) est relié le long de son bord d'ouverture de jambe (12) au côté respectif (23) de la structure (20) et chaque ouverture de jambe (14) est formée à travers le panneau élastique (30).

5. Couche (10) selon la revendication 1, dans laquelle le point à partir duquel commence la ligne de fixation avant (21) est sur le bord avant (15) de la structure (20) et le point à partir duquel commence la ligne de fixation arrière (22) est sur le bord arrière (17) de la structure (20).

6. Couche (10) selon la revendication 1, dans laquelle les points à partir desquels commencent respectivement la ligne de fixation avant (21) et la ligne de fixation arrière (22), sont sur la ligne médiane longitudinale A-A de la structure (20).

7. Couche (10) selon la revendication 1, dans laquelle la structure (20) comporte des bords avant et arrière (15, 17) qui sont délimités par les lignes de fixation avant et arrière respectives (21, 22).

8. Couche (10) selon la revendication 1, dans laquelle un fronçage est réalisé près de chaque ouverture de jambe (14) en fixant un ou plusieurs plis faits dans une partie de la structure (20).

9. Couche (10) selon la revendication 1, dans laquelle les panneaux élastiques (30) ont des parties ayant des forces de rétraction différentes le long de leur largeur.

10. Couche (10) selon la revendication 1, dans laquelle chaque panneau élastique (30) s'étend au delà de la ligne médiane longitudinale A-A pour recouvrir une partie de la moitié opposée de la structure (20) grâce à quoi un panneau élastique (30) recouvre une partie de l'autre panneau élastique (30) en formant ainsi une couche (10) ayant une ouverture de ceinture réduite (13).

11. Couche (10) selon la revendication 1, dans laquelle le bord d'ouverture de jambe (12) du panneau élastique (30) et le bord latéral (23) de la structure (20) sont dirigés en pointe vers l'intérieur, de l'arrière vers l'avant de la couche (10), en formant ainsi une couche (10) ayant une structure (20) plus large à l'arrière qu'à l'avant.

12. Procédé pour fabriquer une couche à enfiler, à jeter après usage (10) ayant une ouverture de ceinture (13) et des ouvertures de jambe (14) comportant une feuille de dessus perméable aux liquides (24), une feuille de fond imperméable aux liquides (26) une âme absorbante (28) et une paire de panneaux élastiques (30), chacun ayant un bord d'ouverture de ceinture (16) et un bord d'ouverture de jambe opposé (12) comprenant les étapes suivantes :
- on interpose et on fixe l'âme absorbante (28) entre la feuille de dessus (24) et la feuille de fond (26) pour assembler une structure absorbante (20) qui comporte une paire de côtés (23) et une ligne médiane longitudinale A-A divisant la structure (20) en deux moitiés et qui comporte la feuille de dessus (24), la feuille de fond (26) et l'âme absorbante (28) intercalée entre elle, où la structure absorbante (20) est divisée en une partie d'entrejambe (7), une partie de ceinture avant (8) et une partie de ceinture arrière (9) et dans laquelle la partie de ceinture avant (8) et la partie de ceinture arrière (9) s'étendent à l'opposé à partir de la partie d'entrejambe (7) le long de la ligne médiane longitudinale A-A,
caractérisé en ce que lesdites étapes consistent en outre :
- à faire coïncider un panneau élastique pré-étiré (30) avec chaque moitié de la structure (20) de telle sorte que le bord d'ouverture de ceinture (16) du panneau élastique (30) s'étende au moins le long de la ligne médiane longitudinale A-A de la structure (20) et
- à fixer le panneau élastique pré-étiré (30) à la structure (20) le long des lignes de fixation (21, 22) qui comportent une ligne de fixation avant (21) reliant un point, à proximité de la ligne médiane longitudinale A-A dans la partie de ceinture avant (8) de la structure (20) à un point sur chaque côté de la structure (20), et une ligne de fixation arrière (22) reliant un point, à proximité de la ligne médiane longitudinale A-A dans la partie de ceinture arrière (9) de la structure (20) à un point sur chaque côté de la structure (20), grâce à quoi les bords d'ouverture de ceinture (16) des panneaux élastiques (30) forment complètement l'ouverture de ceinture (13) de la couche (10).

13. Procédé pour fabriquer une couche à enfiler, à jeter après usage (10) selon la revendication 12,
caractérisé en ce que lesdites étapes consistent en outre :
- à mettre les bords d'ouverture de jambe (12) des panneaux élastiques pré-étirés (30) sur chaque côté de la structure (20) ;
- à relier les panneaux élastiques (30) entre eux avec la feuille de dessus (24) et la feuille de fond (26) le long du bord latéral (23) de la structure (20) pour intégrer les panneaux élastiques (30) et la structure (20);
- à former les ouvertures de jambe (14) dans les parties le long de chaque bord d'ouverture de jambe (12) des panneaux élastiques (30).

14. Procédé pour fabriquer une couche à enfiler, à jeter après usage (10) selon la revendication 12,
caractérisé en ce que lesdites étapes consistent en outre :
- à élastifier les aires latérales de la feuille de dessus (24) pour former les panneaux élastiques (30) ayant un bord latéral (25) ;
- à former les ouvertures de jambe (14) le long d'une ligne de bordure entre la structure (20) et chaque panneau élastique (30);
- à plier les panneaux élastiques (30) le long d'une ligne de bordure de manière à ce que le bord latéral (25) des panneaux élastiques s'étende au moins jusqu'à la ligne médiane longitudinale A-A de la structure (20).

15. Procédé pour préparer une couche (10) selon les revendications 12, 13 ou 14, dans lequel le panneau élastique (30) est replié de manière à ce que les bords d'ouverture de ceinture (16) s'étendent au delà de la ligne médiane longitudinale A-A de la structure (20) pour recouvrir une partie de la moitié opposée de la structure (20), grâce à quoi un panneau élastique (30) recouvre une partie de l'autre panneau élastique (30).
